# EUROPEAN PATENT APPLICATION

(11) **EP 4 477 159 A2**
(43) Date of publication of application: **18.12.2024**
(21) Application number: 24181570.3
(22) Date of filing: 12.06.2024
(51) Int. Cl.: A61B 17/04

(54) **SURGICAL CONSTRUCTS FOR TISSUE FIXATION**

(30) Priority: 14.06.2023 US 202363508001 P
(71) Applicant: Arthrex, Inc, Naples, FL 34108-1945 (US)
(72) Inventor: CUNY, Julia F., Naples, 34108-1945 FL (US); ROY, Andrew D., Naples, 34108-1945 FL (US)
(74) Representative: Lohr, Jöstingmeier & Partner

(57) **Abstract**

Knotless constructs and methods of tissue repairs are disclosed herein.

## Description

### BACKGROUND

The disclosure herein relates to the field of surgery and, more specifically, to knotless suture constructs and associated methods of tissue repairs.

### SUMMARY

Knotless constructs, surgical systems, assemblies, and methods of tissue repairs are disclosed. A construct can create a knotless repair. In an embodiment, a construct as described herein can be self-locking and tensionable. A construct can include fixation devices in the form of soft suture anchors knotlessly attached to a fixed length of flexible coupler. A flexible coupler can be passed through the fixation devices and each limb of the flexible coupler can be spliced back into itself to form a tensionable mechanism.

Methods of tissue repairs are also disclosed. A first tissue is approximated to a second tissue with a surgical construct that includes at least one tensionable construct with a knotless mechanism. Free ends of a flexible coupler (suture or tape) are passed through bodies of fixation devices (all-suture soft anchors). Ends of the flexible coupler are spliced and form adjustable, closed, tensionable, flexible loops that are interconnected. The ends can be pulled to tension and lock the construct.

### BRIEF DESCRIPTION OF THE DRAWINGS

FIGS. 1-3 illustrate a surgical construct.
FIGS. 4-9 illustrate the surgical construct of FIGS. 1-3 employed in tissue repair.
FIG. 10 illustrates a schematic view of another tissue repair.

### DETAILED DESCRIPTION

The disclosure provides surgical adjustable loop constructs, suture loop mechanisms, in-line surgical repairs, and methods for securing a first tissue to a second tissue (for example, bone to bone) with a tensionable construct including adjustable, knotless, flexible, closed loops.

In an embodiment, the disclosure provides an all-suture tissue fixation construct. An orthopedic implant construct can attach or re-attach normal anatomical structures, tissue to tissue, bone to bone, and/or bone to soft tissue. The construct can include a fixed length of a single continuous flexible coupler in the form of suture, round, and/or flat suture attached to at least two fixation devices.

A flexible coupler can be attached in a knotless manner to two soft suture anchors for knotless, in-fine, all-suture fixation. Terminal ends of the flexible coupler exit the fixation devices and are spliced to themselves to form a plurality of knotless, continuous, flexible, closed adjustable loops having an adjustable perimeter. The construct can be shrunk when both terminal ends are pulled. When the terminal ends are pulled, the construct shrinks, *i.e.,* the perimeters of the knotless, continuous, flexible, closed adjustable loops decrease. The construct allows the user (for example, surgeon) to control the tension of the flexible coupler on a first tissue (for example, tissue fragment such as cartilage) to be attached to a second tissue (for example, bone such as condyle).

A tissue repair system includes first and second fixation devices each in the form of a soft suture anchor; and a flexible coupler slidingly attached to the first and second fixation devices, wherein the flexible coupler includes two flexible ends (a first end and a second end) and wherein the first end forms a first splice and a first loop, and the second end forms a second splice and a second loop. The system can further include a suture passer and a shuttle/pull device attached to the flexible coupler. A flexible coupler can be of either round and/or flat design. A flexible coupler can be suture and/or tape. A tissue repair system can consist essentially of suture such as ultrahigh molecular weight polyethylene suture. A tissue repair system can provide knotless, in-line, all-suture tissue fixation.

A construct can be employed to re-attach normal anatomical structures, *i.e.,* a first tissue to a second tissue, such as soft tissue, tendon, ligament, and/or bone, to each other and/or any combination of one another, by employing an in-fine, knotless mechanism. A flexible coupler can be a fixed length of suture and/or tape. A construct can be employed as a stand-alone construct or with additional fixation devices, for example, attached to an additional implant, anchor, screw, plate, button (such as a metal button), etc.

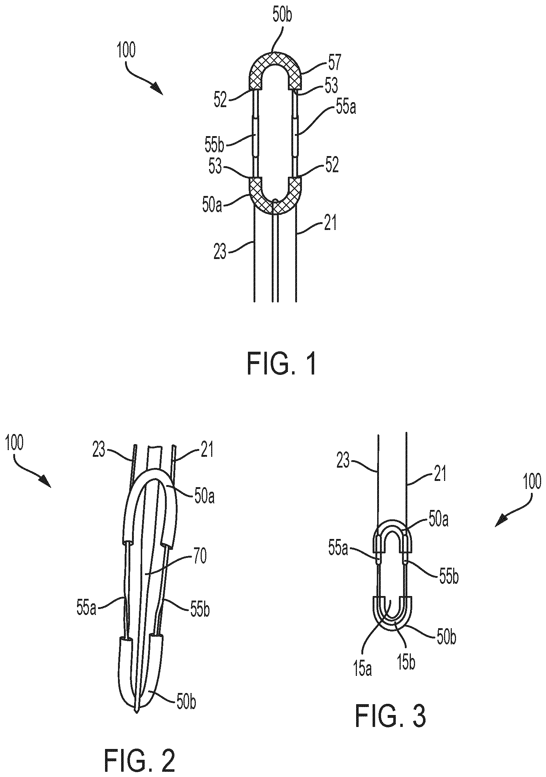

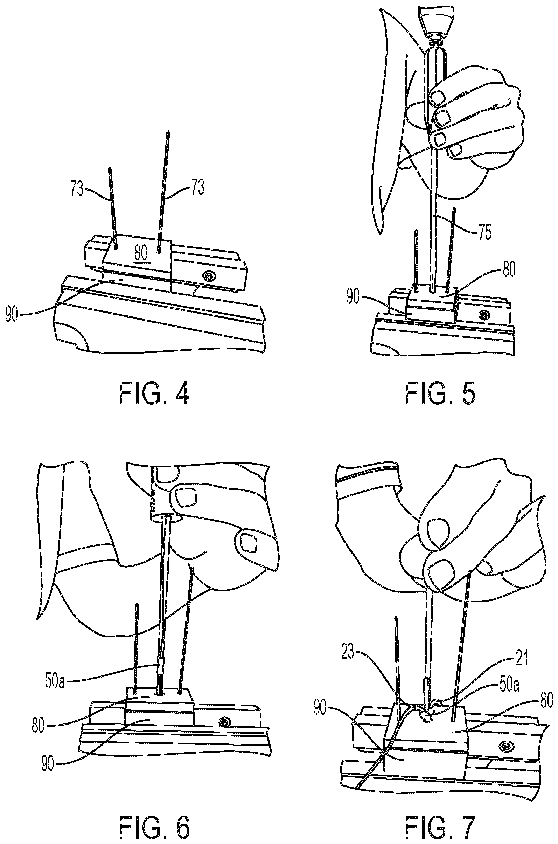

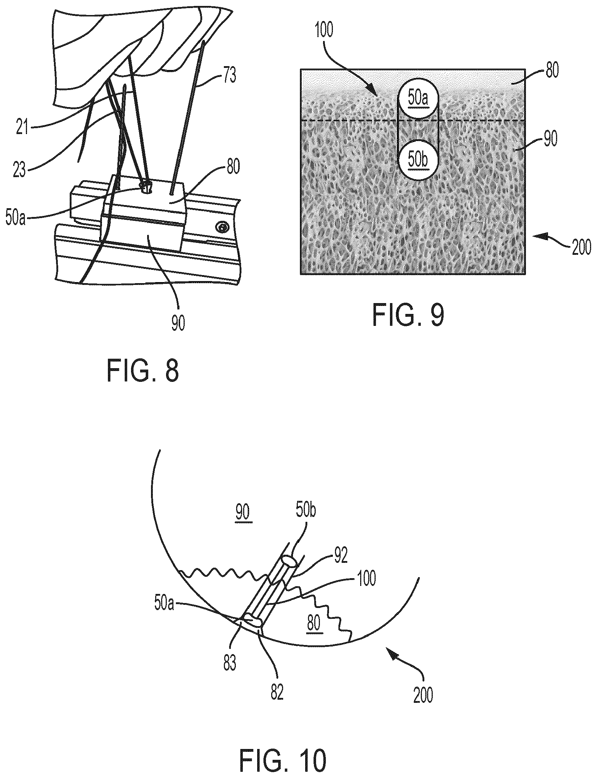

Referring now to the drawings, where like elements are designated by like reference numerals, FIGS. 1-3 illustrate structural elements of surgical assembly 100 (construct 100; surgical construct 100; knotless construct 100; soft anchor cartilage fixation anchor 100; soft anchor cartilage fixation system 100; system 100) formed of fixation devices 50a, 50b connected by flexible coupler 20. FIGS. 4-10 illustrate exemplary steps of tissue repair 200 (FIGS. 9 and 10) with surgical assembly 100.

As shown in FIGS. 1-3, assembly 100 includes two fixation devices 50a, 50b in the form of soft-suture anchors 50a, 50b. As detailed below, soft-suture anchors 50a, 50b are knotlessly attached to flexible coupler 20 by one or more splices and one or more loops. Each fixation device 50a, 50b can be in the form of a soft anchor (soft suture sheath, soft suture anchor, all-suture soft knotless anchor, implant) provided with a soft anchor sleeve 51 (sheath or tubular member 51) with two open ends 52, 53. At least one flexible coupler 20 extends through each of the soft anchor sleeve 51 (sheath 51) of each fixation device 50a, 50b.

Flexible coupler 20 can extend through the anchor sleeves in similar or different directions and/or orientations and/or locations. Flexible coupler 20 can extend through the whole length of each sleeve, *i.e.,* enters one of the two open ends 52, 53 and exits other of the two open ends 52, 53. Details of an exemplary soft suture anchor with a soft anchor sleeve (sheath or tubular member) and flexible shuttling strands are set forth, for example, in US Patent 10,849,734 issued Dec. 1, 2020, entitled "Methods of Tissue Repairs," the disclosure of which is incorporated by reference in its entirety herein.

In an embodiment, fixation devices 50a, 50b can be formed essentially of suture. In an embodiment, fixation devices 50a, 50b can be formed of braided suture that can include fibers of ultrahigh molecular weight polyethylene (UHMWPE). In an embodiment, fixation devices 50a, 50b can be formed of elastic suture. In an embodiment, fixation devices 50a, 50b can be formed of UHMWPE braided with polyester. UHMWPE is easy to splice and pierce; allows the construct to deform and remain in deformed shape; even at high PPI, it has minimal fraying and snagging; and has a low coefficient of friction. Polyester is difficult to splice without fraying suture; allows the construct to retain initial form; is difficult to pierce and pass TightRope^{®} suture without fraying and snagging; and has a high coefficient of friction.

Flexible coupler 20 (tensionable construct 20; coupler 20; flexible material 20; flexible strand 20; flexible tape 20; suture 20) can be formed of one single continuous coupler in form of suture, either round and/or flat suture, for example, a suture strand or suture tape. Flexible coupler 20 is provided with two terminal ends, a first end 21 and a second end 23. Flexible coupler 20 can be a fixed length of suture and/or tape, such as suture tape.

In an embodiment, surgical assembly 100 can be formed by passing one of two terminal ends 21, 23 (for example, first end 21) of the flexible coupler through one of the soft-anchors 50a, 50b (for example, first soft-anchor 50a, or proximal anchor 50a). Terminal end 21 of flexible coupler 20 is passed through at least a portion of body 11 of fixation device 50a so that the two terminal ends 21, 23 of flexible coupler 20 reside outside the body 11 of fixation device 50a.

Each terminal end 21, 23 is then passed through the other of the soft-anchors 50a, 50b (for example, second soft-anchor 50b, or distal anchor 50b). The terminal ends 21, 23 are passed through distal soft anchor 50b in different directions (for example, opposite directions) and each end is spliced through itself. First terminal end 21 forms first splice 55a and first suture loop 15a. Second terminal end 23 forms second splice 55b and second suture loop 15b. The passes and splices can be formed with suture passers and/or shuttle/pull devices and/or suture passing instruments such as needles, FiberLink^{™} loops, nitinol loops, or any suture passing device that includes an eyelet/loop for passing the flexible coupler 20.

Tensionable construct 100 can be shrunk when both terminal ends 21, 23 are pulled to decrease the perimeter of at least one of the flexible, closed, knotless, adjustable loops 15a, 15b. Additional flexible strands can be attached to construct 100 by passing the flexible strands through any of loops 15a, 15b. Additional strands can be FiberWire^{®} suture, TigerWire^{®} suture, FiberTape^{®} suture tape, among many others.

FIG. 2 illustrates assembly 100 loaded onto inserter 70, for example, a forked inserter 70. As noted above, implant 100 consists of two soft suture sheaths 50a, 50b connected by way of a single continuous suture 20 which loops through both before having each limb 21, 23 spliced back into itself to create a means for knotless tensioning. As the two sutures 21, 23 are tensioned, the loops 15a, 15b shrink and decrease the distance between the sheaths, causing compression of the fragment into the condyle. Distal fixation device 50b (distal anchor 50b or distal sheath 50b) is secured on a tip of inserter 70. Distal sheath 50b is inserted into the condyle like existing bunching suture anchors. Proximal fixation device 50b (proximal anchor 50a or proximal sheath 50a) is pulled into a tapered hole 83 created in the fragment using a stepped drill. The proximal sheath 50a does not have to "seat" but rather plugs in the hole.

In an embodiment, system 100 is a soft anchor cartilage fixation anchor. System 100 is a single shot cartilage system repair. In an embodiment, system 100 consists essentially of a TightRope^{®} construct loaded onto (or with) two soft suture anchors. Final fixation of boney fragment is an in-fine, all-suture, knotless fixation.

Reference is now made to FIGS. 4-10 which illustrate steps of an exemplary method of tissue repair with system. The embodiment detailed below is an exemplary cartilage fixation, *i.e.,* a chondral boney fragment fixation conducted in a knotless, all-suture manner for an all-suture fixation. The soft anchor cartilage fixation is a "single shot" cartilage fixation in that the soft suture anchors are provided "in-line" and in a simple manner, with fewer steps, and increased fixation.

### FIG. 4:

Step 1 - For provisional fixation, secure osteochondral defect/flap 80 (first tissue 80; fragment 80) with one or two K-wires 73 such that they will provisionally stabilize the fragment.

### FIG. 5:

Step 2 - Using a drill guide, drill a pilot hole 82 until desired depth is reached depending on thickness of chondral fragment 80. A stepped or tapered drill can be used to form stepped hole 83 (illustrated more clearly in FIG. 10) in chondral fragment 80.

### FIG. 6:

Step 3 - Impact the distal anchor 50b into tunnel/opening/socket 92 in femoral condyle 90 (second tissue 90). Pull back on the strands 21, 23 to set the anchor in bone 90.

### FIG. 7:

Step 4 - Implant the second suture implant 50a into the chondral fragment 80. Implant 50a resides within tapered/stepped hole 83.

### FIG. 8:

Step 5 - Using the suture exiting the condyle, tension down the construct 100 to reduce the fragment 80 back to bone 90 and achieve final repair 200 (FIG. 9). Once desired tension is achieved, ends 21, 23 can be cut to achieve a knotless, in-line repair. Final repair 200 is also schematically illustrated in FIG. 10. Openings 92, 82, 83 house fixation devices 50a, 50b and splices 55a, 55b of flexible, adjustable, tensionable, self-locking loops 15a, 15b.

Construct 100 can be employed with one or more biologics, for example, bone-to-bone biologics. Various medicinal and/or therapeutic agents, for example, antiseptics, antibiotics, drugs, pharmaceutical agents, hormones, and growth materials (for example, autogenous growth factors such as platelet-rich plasma (PRP), or autologous factors) among many others can be added at the repair site to aid in the wound closure and overall healing.

The benefits of construct 100 to the patient include no prominent metal hardware left in the body that can cause soft tissue irritation; less scar tissue formation; and immediate enclosure of the bone tunnel allowing faster healing. Construct 100 eliminates the large "knots" palpable under the skin of the prior art designs, allowing for specific tailoring of height relative to the bone surface.

Construct 100 of FIGS. 1-10 is an adjustable, tensionable loop construct. Construct 100 is an all-inside, self-locking, adjustable, knotless construct. Construct 100 can be a suture/soft anchors construct in the form of an orthopedic implant construct which may be utilized to attach or re-attach a first tissue to a second tissue, for example, normal anatomical structures, bone to bone, tissue to tissue, and/or bone to tissue, among others. The construct includes a single strand of suture run through two soft suture anchors, with ends of the suture threaded back through itself (interwoven back through itself) to create a tensionable, self-locking, adjustable loop, anchoring construct. The construct can be provided assembled, disassembled, as part of a kit, with or without additional instruments (such as suture passers), fixation devices and/or flexible couplers, to facilitate passing through tunnels and/or bone sockets and/or openings.

All-inside, suture-based, arthroscopic tissue repair devices/constructs of the present disclosure can be employed for any tissue positioning and/or tissue adjustment applications, for example, in fixation of bone to bone (such as small joint applications, or acromioclavicular joint fixation techniques) which employ two fixation devices (for example, two flexible suture anchors) joined by a continuous suture loop formed by a continuous flexible coupler. In exemplary embodiments only, construct 100 of the present disclosure can be employed in a method of bunion repair and/or in a method of Lisfranc repair. Similarly, construct 100 can be employed in any method of fixation of bone.

The suture implant with the self-locking mechanism can be utilized in surgical procedures such as rotator cuff repair, Achilles tendon repair, patellar tendon repair, ACL/PCL reconstruction, hip and shoulder reconstruction procedures, AC joint reconstruction, syndesmosis reconstruction, quad/patellar tendon rupture repair, hallux-valgus repair, proximal and/or distal biceps tendon repair, humerus and radius repair, and any other tendon repair to bone, among many others, all conducted in a knotless manner.

A surgical assembly 100 includes a plurality of fixation devices 50a, 50b and a knotless, adjustable, self-locking tensionable construct 20 loaded on the fixation devices 50a, 50b. The tensionable construct 20 includes at least one flexible coupler 20 having a first end 21 and a second end 23; first and second closed, adjustable, continuous, flexible loops 15a, 15b; and first and second splices 55a, 55b. Fixation devices 50a, 50b are all-suture soft anchors. Surgical assembly 100 can connect first tissue 80 to second tissue 90. First tissue 80 can be a bone fragment or osteochondral fragment; and second tissue 90 can be bone. Distal fixation device 50b can reside and be secured within opening 92 formed within second tissue 90. Proximal fixation device 50a can reside and be secured within openings 82, 83 of first tissue 80. Terminal ends 21, 23 are pulled to decrease the distance between the two fixation devices 50a, 50b and decrease the length and perimeter of the flexible, adjustable, closed, knotless loops 15a, 15b. Surgical assembly 100 can consist essentially of suture. Surgical assembly 100 can consist essentially of elastic suture.

A method of tissue repair 200 comprises *inter alia:* passing a flexible coupler 20 through sheaths of two fixation devices 50a, 50b, to form a knotless, tensionable, self-locking, adjustable construct with two loops 15a, 15b and two splices 55a, 55b; securing one of the two fixation devices 50a, 50b in a first tissue 90; and securing the other of the two fixation devices 50a, 50b in a second tissue 80. The method can further include tensioning the construct by pulling on terminal ends 21, 23 of flexible coupler 20.

A method of knotless repair 200 comprises: attaching a single, continuous, flexible coupler 20 with a first end 21 and a second end 23 to a first fixation device 50a by passing one end 21 of the first and second ends 21, 23 through the first fixation device 50a with a first passing device, wherein the other end 23 of the first and second ends 21, 23 is attached to a second passing device; passing each of the first end 21 and second end 23 through a second fixation device 50b and then through each of the first end 21 and second end 23 to form a plurality of flexible, continuous, closed, adjustable, knotless loops 15a, 15b and splices 55a, 55b; securing second fixation device 50b in a first tissue 90; securing first fixation device 50a in a second tissue 80; and pulling on the first end 21 and the second end 23 to approximate the second tissue 80 to the first tissue 90. The first tissue 90 can be bone, and the second tissue 80 can be bone or osteochondral fragment.

Flexible coupler 20 can be formed of various flexible materials and strands such as round suture, flat suture, ribbon, or flat tape (for example, suture tape) or combination of suture and tape. Exemplary materials can include suture, silk, cotton, nylon, polypropylene, polyethylene, ultrahigh molecular weight polyethylene (UHMWPE), polyethylene terephthalate (PET), and polyesters and copolymers thereof, or combinations thereof. Flexible strand/coupler 20 can have cross-sections of various forms and geometries, including round, oval, rectangular, or flat, among others, or combination of such forms and geometries. In an exemplary embodiment only, flexible coupler 20 can be formed of a high strength suture material such as FiberWire^{®} suture, sold by Arthrex, Inc. of Naples, Fla., and described in US 6,716,234, the disclosure of which is incorporated by reference herein. FiberWire^{®} suture is formed of an advanced, high-strength fiber material, namely ultrahigh molecular weight polyethylene (UHMWPE), sold under the tradenames Spectra^{®} (Honeywell International Inc., Colonial Heights, Va.) and Dyneema^{®} (DSM N.V., Heerlen, the Netherlands), braided with at least one other fiber, natural or synthetic, to form lengths of suture material. Flexible coupler 20 can be braided or multi-filament suture such as FiberTape^{®} suture tape (as disclosed in US 7,892,256, the disclosure of which is incorporated in its entirety herewith). If suture tape is employed, the tape can have sections with different tapers (for example, 2 or 3 sections of gradual tapers or gradual widths) to facilitate easy formation of the splice regions 55a, 55b and loops 15a, 15b. For example, splice region 55a, 55b can be round suture while loops 15a, 15b can be formed of flat sections.

Flexible coupler 20 can be also formed of a stiff material, or combination of stiff and flexible materials, particularly for the regions of the couplers that are passed/spliced through the body of the coupler and depending on whether they are employed with additional fixation devices. Various regions, or sections of flexible coupler 20 can be coated and/or provided in different colors for easy manipulation during the surgical procedure. Flexible coupler 20 can be provided with tinted tracing strands, or otherwise contrast visually with the sheath 51 of the soft suture anchors 50a, 50b, which remains a plain, solid color, or displays a different tracing pattern, for example. Easy identification of suture *in situ* is advantageous in surgical procedures, particularly during arthroscopic surgeries, endoscopic and laparoscopic procedures.

Various elements of construct 100 can be also coated (partially or totally) with wax (beeswax, petroleum wax, polyethylene wax, or others), silicone (Dow Corning silicone fluid 202A or others), silicone rubbers (Nusil Med 2245, Nusil Med 2174 with a bonding catalyst, or others) PTFE (Teflon, Hostaflon, or others), PBA (polybutylate acid), ethyl cellulose (Filodel) or other coatings, to improve lubricity of the suture or tape, knot security, pliability, handleability or abrasion resistance, for example. If desired, at least a region of the flexible strands/coupler 20 can be coated, impregnated, or otherwise stiffened with a material such as plastic, for example.

The term "high strength suture" is defined as any elongated flexible member, the choice of material and size being dependent upon the particular application. For the purposes of illustration and without limitation, the term "suture" as used herein may be a cable, filament, thread, wire, fabric, or any other flexible member suitable for tissue fixation in the body.

## Claims

1. A surgical construct comprising a flexible coupler knotlessly connected to a first soft anchor and to a second soft anchor.

2. The surgical construct of claim 1, wherein the flexible coupler passes at least once through a first tubular sheath of the first soft anchor and at least once through a second tubular sheath of the second soft anchor to form at least one closed, continuous, flexible loop with an adjustable perimeter.

3. The surgical construct of claim 2, wherein the flexible coupler enters the first tubular sheath at a first location, extends within the first tubular sheath and exits the first tubular sheath at a second location, wherein the second location is different from the first location.

4. The surgical construct of claim 3, wherein the flexible coupler enters the second tubular sheath at a first location, extends within the second tubular sheath and exits the second tubular sheath at a second location, wherein the second location is different from the first location.

5. The surgical construct of any of the previous claims, wherein the flexible coupler is spliced through itself at one or more locations.

6. The surgical construct of claim 5, wherein the flexible coupler is spliced through itself at two separate locations.

7. The surgical construct of any of the previous claims, wherein each of the first soft anchor and the second soft anchor is an all-suture anchor, and the flexible coupler is round suture.

8. The surgical construct of any of the previous claims, wherein the construct includes suture, and/or wherein the flexible coupler includes elastic material.

9. A surgical assembly for tissue repairs, comprising:
a first fixation device and a second fixation device, each including suture; and
a flexible coupler passed through the first fixation device and
through the second fixation device, the flexible coupler forming in a knotless manner at least one splice and at least one continuous, adjustable, tensionable loop extending between the first fixation device and the second fixation device.

10. The surgical assembly of claim 9, wherein each of the first fixation device and the second fixation device includes a tubular sheath with two open ends.

11. The surgical assembly of claim 9 or 10, wherein the flexible coupler forms two splices and two continuous, adjustable, tensionable loops extending between the first fixation device and the second fixation device.

12. The surgical assembly of claim 11, wherein, when ends of the flexible coupler are pulled, a perimeter and a length of each of the two continuous, adjustable, tensionable loops decreases and tensions the surgical assembly.

13. The surgical assembly of any of claims 9 to 12, wherein the flexible coupler is suture or suture tape.

14. The surgical assembly of any of claims 9 to 12, wherein the tissue repair is rotator cuff repair, AC joint repair, syndesmosis repair, Achilles tendon repair, patellar tendon repair, ACL/PCL reconstruction, hip and shoulder reconstruction, AC joint reconstruction, syndesmosis reconstruction, quad/patellar tendon rupture repair, or hallux-valgus repair.

15. The surgical assembly of any of claims 9 to 12, wherein the tissue repair is osteochondral repair.
